# EUROPÄISCHE PATENTSCHRIFT

(11) **EP 0 649 833 B1**
(45) Veröffentlichungstag und Bekanntmachung des Hinweises auf die Patenterteilung: **28.02.1996**
(21) Anmeldenummer: 94116049.1
(22) Anmeldetag: 12.10.1994
(51) Int. Cl.: C07C 221/00, C07C 225/34

(54) **Verfahren zur Herstellung von Anthrachinonamin-Verbindungen**
Process for the preparation of anthraquinonamine compounds
Procédé pour la préparation de composés d'amines d'anthraquinone

(30) Priorität: 21.10.1993 DE 4335975
(43) Veröffentlichungstag der Anmeldung: 26.04.1995
(73) Patentinhaber: BASF Aktiengesellschaft, D-67063 Ludwigshafen (DE)
(72) Erfinder: Bergmann, Udo, Dr., D-49477 Ibbenbüren (DE); Hoch, Helmut, Dr., D-67273 Weisenheim (DE); Kilburg, Heike, Dr., D-67346 Speyer (DE); Kohlhaupt, Reinhold, Dr., D-67227 Frankenthal (DE); Niedenbrueck, Matthias, Dr., D-67117 Limburgerhof (DE)

(56) Entgegenhaltungen:
- EP-A- 0 047 205
- EP-A- 0 136 981
- EP-A- 0 199 670
- EP-A- 0 326 866
- EP-A- 0 375 990
- EP-A- 0 382 053

## Beschreibung

Die vorliegende Erfindung betrifft ein neues Verfahren zur Herstellung von Anthrachinonaminverbindungen durch Kondensation von mindestens eine primäre Aminogruppe enthaltenden Anthrachinonverbindungen mit halogenierten Aromaten oder von halogenierten Anthrachinonverbindungen mit primären aromatischen Aminen in Gegenwart eines Kupferkatalysators und eines säurebindenden Mittels in einem organischen Lösungsmittel.

Anthrachinonamine stellen wichtige Vorprodukte für Küpenfarbstoffe dar oder sind bereits selbst bekannte Küpenfarbstoffe, die vielfach zum Färben oder Bedrucken von Baumwolle oder Baumwollmischgeweben verwendet werden.

Die Herstellung der Anthrachinonamine erfolgt bekanntermaßen durch Kondensation eines durch mindestens eine primäre Aminogruppe substituierten Anthrachinons mit einem entsprechenden halogenierten aromatischen Reaktionspartner, wobei dem Reaktionsgemisch üblicherweise ein Kupferkatalysator und ein säurebindendes Mittel zugesetzt werden.

Allgemein bekannte Lösungsmittel für diese Umsetzung sind Naphthalin (BIOS Final Report 987, Seite 71 und 76 sowie 1493, Seite 32, DRP 696 425) und Nitrobenzol (BIOS Final Report 987, Seite 63, FIAT Final Report 1313 II, Seite 102 und 182, FR-A-654 536, CH-A-208 953, US-A-3 040 063).

Diese bislang verwendeten Lösungsmittel zeigen jedoch eine Reihe von Nachteilen: Naphthalin sublimiert bei den für die Kondensation benötigten hohen Temperaturen, weshalb bei den Syntheseapparaturen ein hoher Reinigungsaufwand erforderlich ist, um technische Störungen, z.B. durch Verstopfung von Leitungen, zu vermeiden. Außerdem haftet Naphthalin den Reaktionsprodukten stark an und muß technisch aufwendig durch Wasserdampfdestillation entfernt werden.

Kondensationsreaktionen in Nitrobenzol erfordern vielfach lange Reaktionszeiten, um einen vollständigen Umsatz zu erreichen, was oftmals trotzdem nicht gelingt, so daß die Endprodukte durch nicht umgesetzte Edukte und weitere Nebenprodukte verunreinigt sind und, wenn überhaupt möglich, aufwendigen Reinigungsmethoden unterzogen werden müssen oder zu Endfarbstoffen mit verschlechterter Coloristik führen.

Es werden daher verschiedene Vorgehensweisen vorgeschlagen, um den genannten Problemen bei der Synthese in Nitrobenzol abzuhelfen. So wird in der EP-A-119 956 eine portionsweise Zudosierung des Katalysators während der Reaktion bei 195-205°C und in der EP-A-136 981 eine thermische Nachbehandlung der Reaktionsmischungen bei 215-250°C unter Druck beschrieben. Nach der EP-A-199 670 erweist es sich schließlich als vorteilhaft, die Edukte zunächst bei Raumtemperatur in Nitrobenzol zu suspendieren und dann so in weiteres, auf Reaktionstemperatur erhitztes Nitrobenzol einzutragen, daß die Temperatur von etwa 210°C gehalten werden kann. Diese Verfahrensweisen vermögen jedoch ebenfalls nicht zu befriedigen, da sie erhöhten technischen Aufwand (zusätzliche Kessel, Zudosierung bei Siedetemperatur, Druckapparatur) erfordern.

Nicht zuletzt liegt ein besonderer Nachteil bei der Verwendung von Nitrobenzol auch in dessen gesundheitsgefährdender Wirkung, die eine aufwendige Absicherung der Reaktionsapparatur und die restlose Entfernung des Nitrobenzols aus den Produkten notwendig macht, was lange Behandlungszeiten erfordert.

Der Erfindung lag daher die Aufgabe zugrunde, den genannten Mängeln abzuhelfen.

Demgemäß wurde ein Verfahren zur Herstellung von Anthrachinonaminverbindungen durch Kondensation von mindestens eine primäre Aminogruppe enthaltenden Anthrachinonverbindungen mit halogenierten Aromaten oder von halogenierten Anthrachinonverbindungen mit primären aromatischen Aminen in Gegenwart eines Kupferkatalysators und eines säurebindenden Mittels in einem organischen Lösungsmittel gefunden, welches dadurch gekennzeichnet ist, daß man als organisches Lösungsmittel einen Benzoesäurealkylester verwendet.

Allgemein kommt es bei den erfindungsgemäß als Lösungsmittel eingesetzten Benzoesäurealkylestern auf die Art des Alkylrestes nicht an, sofern die Ester unter den Reaktionsbedingungen flüssig sind. Geeignet sind insbesondere Benzoesäure-C₁-C₆-alkylester wie Benzoesäurehexyl-, -n-, -iso- und -sek.-pentyl-, -n-, -iso- und -sek.-butyl- und -n- und -isopropylester, bevorzugt Benzoesäureethylester und besonders bevorzugt Benzoesäuremethylester. Es können auch Mischungen der Benzoesäurealkylester verwendet werden.

Die Menge an Lösungsmittel beträgt in der Regel 1 bis 15, vorzugsweise 3 bis 10 kg pro kg Kondensationsprodukt.

Besondere Bedeutung hat das erfindungsgemäße Verfahren zur Herstellung von Anthrachinonaminverbindungen der allgemeinen Formel I 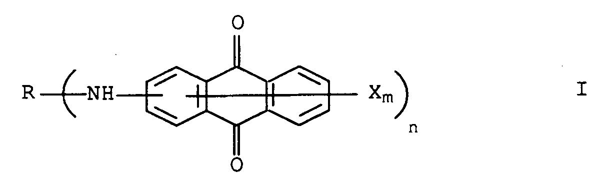
Der Rest R bedeutet insbesondere solche aromatischen Reste, wie sie für Küpenfarbstoffe üblich sind. Im einzelnen seien z.B. Anthrachinon-, Benzanthron-, Anthanthron-, Pyranthron-, Dibenzanthron- (≙ Violanthron-), Isodibenzanthron-, Anthrachinonacridon- (≙ Phthaloylacridon-), Flavanthron-, Fluoranthen- und Dibenzpyrenchinonreste genannt. Geeignet sind ebenfalls Reste von Kondensationsprodukten dieser Aromaten, beispielsweise mit Aminodibenzanthron kondensiertes Halogenpyranthron.

Der Rest R kann für Küpenfarbstoffe übliche Substituenten tragen. Diese entsprechen im wesentlichen den Substituenten X an den Anthrachinonresten.

Geeignete Reste X sind z.B. Halogen, vor allem Chlor und Brom, Hydroxy, Amino, C₁-C₄-Alkylamino, vor allem Methylamino und Ethylamino, C₁-C₄-Alkanoylamino, vor allem Acetylamino und Propionylamino, Benzoylamino, durch Halogen oder C₁-C₄-Alkyl substituiertes Benzoylamino wie p-Chlorbenzoylamino und p-Methylbenzoylamino, und C₁-C₄-Alkyl, vor allem Methyl und Ethyl.

Die Anthrachinonreste können bis zu 4 dieser Substituenten tragen, bevorzugt sind bis zu 2 Substituenten.

Die Variable n bedeutet schließlich ganze Zahlen von 1 bis 4 und hängt von der Art des aromatischen Restes R ab.

Die Herstellung der Anthrachinonamine kann sowohl durch Kondensation eines durch mindestens eine primäre Aminogruppe substituierten Anthrachinons mit einem halogenierten Aromaten als auch durch Kondensation eines halogenierten Anthrachinons mit einem primären aromatischen Amin erfolgen. In der Regel wird die erste Variante bevorzugt sein.

Als Beispiele für bevorzugte Aminkomponenten seien neben 2-Aminoanthrachinon, 2-Amino-3-bromanthrachinon, 2-Amino-3-methylanthrachinon und 2-Amino-3-hydroxyanthrachinon vor allem 1-Aminoanthrachinon, 1,4- und 1,5-Diaminoanthrachinon, 1-Amino-4-benzoylaminoanthrachinon und 1-Amino-5-benzoylaminoanthrachinon sowie Aminoviolanthron genannt.

Bevorzugte Halogenkomponenten, d.h. in der Regel chlorierte und/oder bromierte Verbindungen, sind beispielsweise 1-Chloranthrachinon, 1,4- und 1,5-Dichloranthrachinon, 3-Brom- und 3-Chlorbenzanthron, 3,9-Dibrom- und 3,9-Dichlorbenzanthron, Bromdichloranthrachinonacridon, Bromanthrachinonacridon, Dibromfluoranthen, Tetrabrompyranthron und Dibromviolanthron.

Die Aminkomponente, gewünschtenfalls auch ein Gemisch verschiedener Aminkomponenten, und die Halogenkomponente werden bevorzugt in stöchiometrischen Mengen eingesetzt; ein Überschuß von bis zu etwa 20 % einer der Komponenten ist jedoch auch möglich.

Die Kondensationsreaktion wird zweckmäßigerweise in Gegenwart eines Kupferkatalysators und eines säurebindenden Mittels durchgeführt.

Als Katalysator geeignet sind Kupferverbindungen wie Kupfer(I)acetat, Kupfer(I)bromid, Kupfer(I)chlorid, bevorzugt Kupfer(I)oxid und besonders bevorzugt Kupferpulver durchgeführt. In der Regel kommen pro kg Kondensationsprodukt 5 bis bis 150 g Katalysator zum Einsatz.

Geeignete säurebindende Mittel sind z.B. Magnesiumoxid, Calciumoxid und -hydroxid, Natriumacetat, Natriumdihydrogen- und Dinatriumhydrogenphosphat und Kaliumdihydrogen- und Dikaliumhydrogenphosphat, besonders Kaliumcarbonat und -phosphat und ganz besonders Natriumcarbonat und Natriumphosphat. Üblicherweise werden pro kg Kondensationsprodukt 0,15 bis 1 kg säurebindendes Mittel eingesetzt.

In einigen Fällen, z.B. bei der Umsetzung von Tetrabrompyranthron, empfiehlt sich auch die Anwesenheit eines Oxidationsmittels. Hierfür kommen insbesondere aromatische Nitroverbindungen in Betracht, z.B. o-, m- und p-Nitrobenzoesäure, o-, m- und p-Nitrobenzoesäuremethyl- und -ethylester, o-, m- und p-Nitrochlorbenzol, o-, m- und p-Nitrophenol sowie o-, m- und p-Nitrobenzolsulfonsäure und deren Natriumsalze. Geeignete Mengen an Oxidationsmittel betragen im allgemeinen 0,1 bis 0,3 kg pro kg Kondensationsprodukt.

Die erfindungsgemäße Kondensationsreaktion wird im allgemeinen bei 150 bis 250°C durchgeführt. Temperaturen oberhalb des Siedepunkts des Reaktionsgemisches werden dabei in geschlossenen Apparaturen erreicht. Bevorzugt wird jedoch bei 190 bis 210°C und Normaldruck unter Rückfluß des Lösungsmittels gearbeitet.

Die Reaktionszeiten bis zum vollständigen Umsatz betragen beim erfindungsgemäßen Verfahren in der Regel 1 bis 40 h, insbesondere 3 bis 20 h.

Verfahrenstechnisch geht man zweckmäßigerweise so vor, daß man den Benzoesäurealkylester vorlegt, die Edukte, den Katalysator und das säurebindende Mittel sowie gegegebenenfalls das Oxidationsmittel einträgt und die Mischung dann unter Rühren auf die gewünschte Temperatur erhitzt und bis zur beendeten Umsetzung bei dieser Temperatur hält.

Die Aufarbeitung des Reaktionsgemisches auf die Produkte nach Abkühlen kann auf verschiedene Weise erfolgen. Eine Möglichkeit ist die Wasserdampfdestillation des Gesamtansatzes zur Entfernung und Rückgewinnung des Lösungsmittels durch Phasentrennung und Filtration des Produktes aus der wäßrigen Suspension. Eine weitere Möglichkeit besteht darin, das Lösungsmittel, gegebenenfalls im Vakuum, abzudestillieren, den Rückstand in Wasser anzuschlämmen, das Restlösungsmittel durch Wasserdampfdestillation zu entfernen und rückzugewinnen sowie das Produkt wieder aus der wäßrigen Suspension abzufiltrieren. Schließlich kann man das Produkt auch direkt aus dem Reaktionsgemisch abfiltrieren, dann in Wasser anschlämmen, einer Wasserdampfdestillation zur Entfernung und Rückgewinnung des anhaftenden Lösungsmittels unterziehen und schließlich wieder aus der wäßrigen Suspension abfiltrieren.

Das erfindungsgemäße Verfahren zur Herstellung von Anthrachinonaminen zeichnet sich gegenüber den bekannten Verfahren durch einen glatten Reaktionsverlauf aus, Hilfsmaßnahmen wie eine thermische Nachbehandlung des Reaktionsgemisches unter Druck sind nicht erforderlich. Es werden vollständige Umsetzungen erzielt, weshalb die erhaltenen Kondensationsprodukte von hoher Reinheit sind und keine Verunreinigungen durch Edukte und Nebenprodukte aufweisen und direkt zum Färben bzw. zur weiteren Umsetzung zum Farbstoff verwendet werden können. Weiterhin erfolgt die Umsetzung in vielen Fällen deutlich schneller (bis Faktor 2) als in Nitrobenzol.

Das erfindungsgemäße Verfahren stellt also eine vorteilhafte wirtschaftliche, technisch leicht zu verwirklichende und die Umwelt nicht belastende Möglichkeit zur Herstellung von Anthrachinonaminen dar.

### Beispiele

### Herstellung verschiedener Anthrachinonamine

### Beispiel 1

Ein Gemisch aus 500 g Benzoesäuremethylester, 62 g 3-Brombenzanthron, 49 g 1-Aminoanthrachinon, 32 g wasserfreiem Natriumcarbonat und 0,9 g Kupferpulver wurde unter Rühren auf 200°C erhitzt und 6 h bei dieser Temperatur gehalten.

Nach Abkühlen auf Raumtemperatur wurde das Lösungsmittel mit Wasserdampf vollständig abdestilliert. Filtration der wäßrigen Producktsuspension und Trocknung ergaben 107 g des Anthrachinonamins der Formel 1 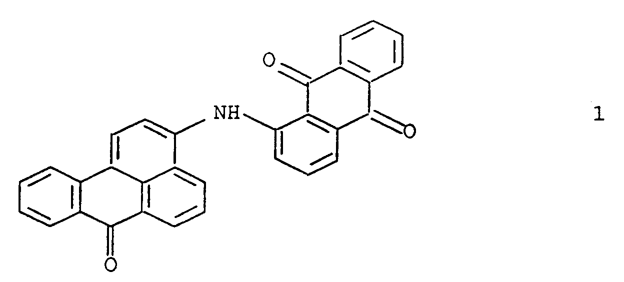
Durch Acridin-Ringschluß in einer Alkalischmelze wurde aus dem Anthrachinonamin 1 auf übliche Weise der Farbstoff C.I. Vat Green 3 mit besonders hoher Farbstärke erhalten, der Baumwolle in sehr guten Echtheiten färbte.

### Beispiel 2

Ein Gemisch aus 530 g Benzoesäuremethylester, 65 g 3,9-Dibrombenzanthron, 77 g 1-Aminoanthrachinon, 27 g wasserfreiem Natriumcarbonat und 1,0 g Kupferpulver wurde unter Rühren auf 200 bis 205°C erhitzt und 22 h bei dieser Temperatur gehalten.

Der auf Raumtemperatur abgekühlte Reaktionsansatz wurde dann in einen Schaufeltrockner überführt und das Lösungsmittel im Vakuum abdestilliert. Der Rückstand wurde in Wasser suspendiert, durch Wasserdampfdestillation vom Restlösungsmittel befreit, abfiltriert und getrocknet. Es wurden 139 g des Anthrachinonamins der Formel 2 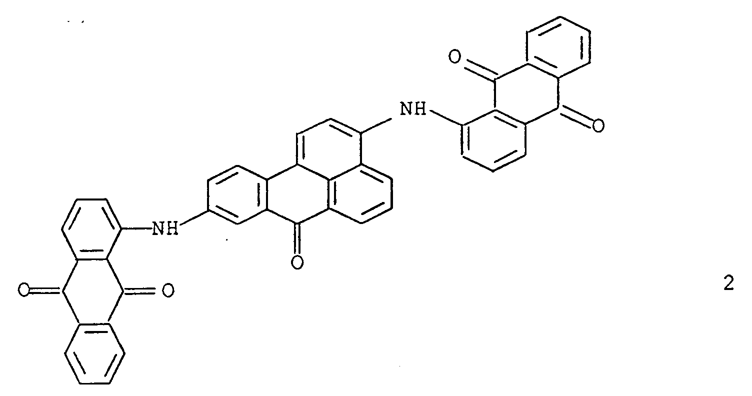
erhalten.

Das Anthrachinonamin 2 lieferte nach alkalischem Acridin-Ringschluß den Farbstoff C.I. Vat Black 25 in guter Qualität.

### Beispiel 3

Ein Gemisch aus 700 g Benzoesäuremethylester, 100 g 3-Brombenzanthron, 80,6 g 1,5-Diaminoanthrachinon, 45 g wasserfreiem Natriumcarbonat und 1,5 g Kupferpulver wurde unter Rühren 4 h auf 200°C erhitzt.

Nach Abkühlen wurde das Lösungsmittel durch Wasserdampfdestillation vollständig entfernt. Filtration und Trocknung ergaben 158 g des Anthrachinonamins der Formel 3 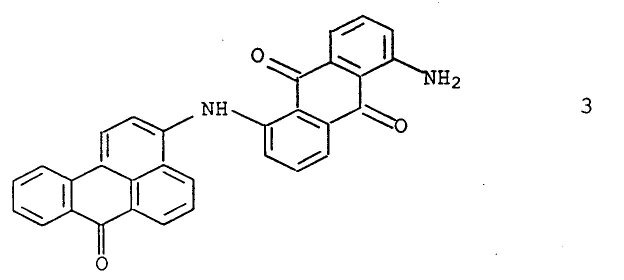
Durch Acridin-Ringschluß und anschließende Benzoylierung der Aminogruppe wurde der entsprechende olivfarbene Küpenfarbstoff mit sehr hoher Farbstärke erhalten.

Die Nacharbeitung von Beispiel 3 in Nitrobenzol ergab dagegen einen Farbstoff mit 40 % geringerer Farbstärke.

### Beispiel 4

Ein Gemisch aus 550 g Benzoesäuremethylester, 66 g 1-Chloranthrachinon, 61 g 1-Aminoanthrachinon, 26 g wasserfreiem Natriumcarbonat und 1,5 g Kupfer(I)chlorid wurde unter Rühren 6 h auf Rückflußtemperatur erhitzt.

Nach Abkühlen, Entfernen des Lösungsmittels durch Wasserdampfdestillation, Filtrieren und Trocknen wurden 116 g des Anthrachinonamins der Formel 4 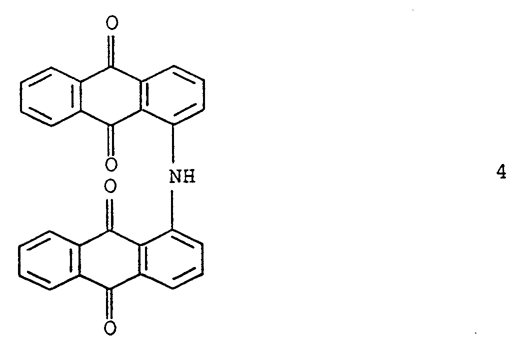
erhalten.

Das Anthrachinonamin 4 konnte zu einem Küpenfarbstoff C.I. Vat Black 27 guter Qualität weiterverarbeitet werden.

### Beispiel 5

Ein Gemisch aus 240 g Benzoesäuremethylester, 33,7 g 1-Chloranthrachinon, 15,7 g 1,4-Diaminoanthrachinon, 7,6 g wasserfreiem Natriumacetat, 7,3 g wasserfreiem Natriumcarbonat und 0,5 g Kupferpulver wurde unter Rühren 6 h auf Rückflußtemperatur erhitzt.

Nach Abkühlen auf Raumtemperatur wurde der Reaktionsansatz filtriert, der Rückstand in Wasser angerührt und anhaftendes Lösungsmittel mit Wasserdampf entfernt. Filtrieren und Trocknen ergaben 35 g des Trianthrachinonamins der Formel 5 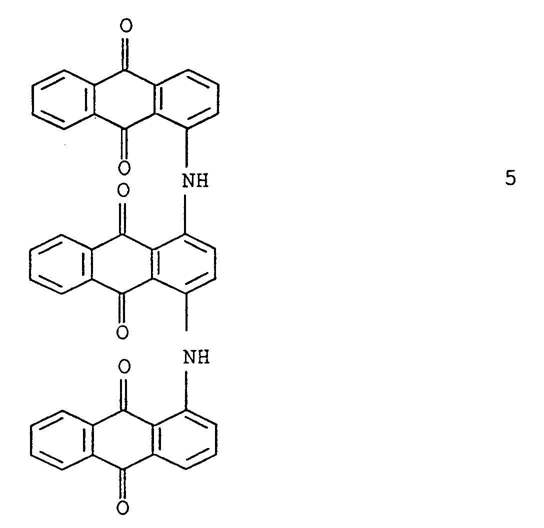
Das Trianthrachinonamin 5 ist Vorstufe des Küpenfarbstoffs C.I. Vat Brown 1.

### Beispiel 6

Ein Gemisch aus 1890 g Benzoesäuremethylester, 132 g 1,5-Dichloranthrachinon, 195 g 1-Aminoanthrachinon, 90 g wasserfreiem Natriumcarbonat und 2,5 g Kupferpulver wurde unter Rühren 8 h auf 200°C erhitzt.

Nach Wasserdampfdestillation zur Entfernung des Lösungsmittels, Filtration und Trocknen wurden 325 g des Trianthrachinonamins der Formel 6 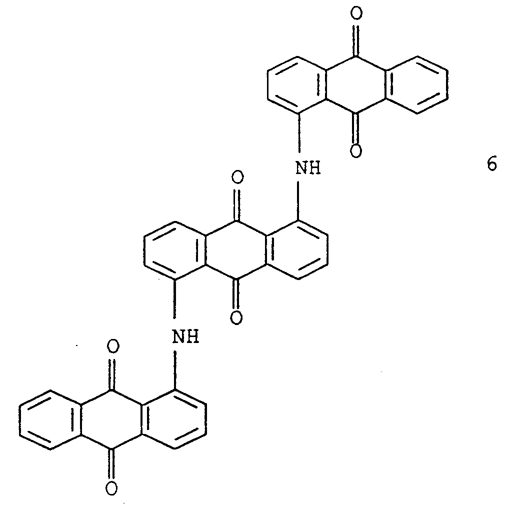
erhalten.

Durch Carbazolierung wurde der Küpenfarbstoff C.I. Vat Orange 11 in guter Qualität hergestellt.

### Beispiel 7

Ein Gemisch aus 740 g Benzoesäuremethylester, 80,0 g Bromdichloranthrachinonacridon, 56,3 g 1-Amino-5-benzoylaminoanthrachinon, 37,5 g wasserfreiem Natriumcarbonat und 1,7 g Kupferpulver wurde unter Rühren 4 h auf 190°C erhitzt und danach in einen Schaufeltrockner überführt.

Nach Abdestillation des Lösungsmittels im Vakuum wurde der Rückstand in Wasser angeschlämmt, mit Wasserdampfdestillation von Lösungsmittelresten befreit, abfiltriert und getrocknet.

Es wurden 105 g des Anthrachinonamins der Formel 7 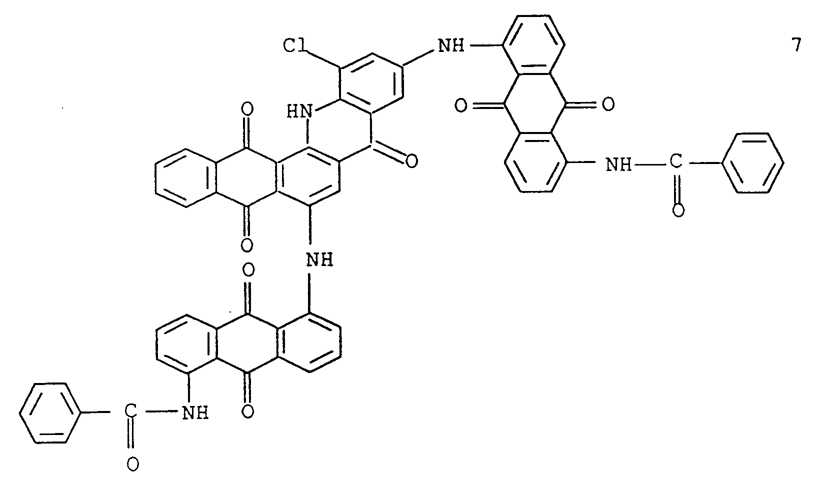
erhalten.

Nachfolgende Carbazolierung in Schwefelsäure lieferte den hochlichtechten Küpenfarbstoff C.I. Vat Brown 55 in sehr guter coloristischer Qualität.

Bei Verwendung von Nitrobenzol anstelle von Benzoesäuremethylester betrug die Reaktionszeit bis zu 8 h, oftmals konnte auch kein vollständiger Umsatz erzielt werden.

### Beispiel 8

Ein Gemisch aus 807 g Benzoesäuremethylester, 72,2 g Bromanthrachinonacridon, 67,2 g 1-Amino-5-benzoylaminoanthrachinon, 46 g wasserfreiem Natriumcarbonat und 1,7 g Kupferpulver wurde unter Rühren 12 h auf 190°C erhitzt.

Aufarbeitung analog Beispiel 7 ergab 123 g des Anthrachinonamins der Formel 8 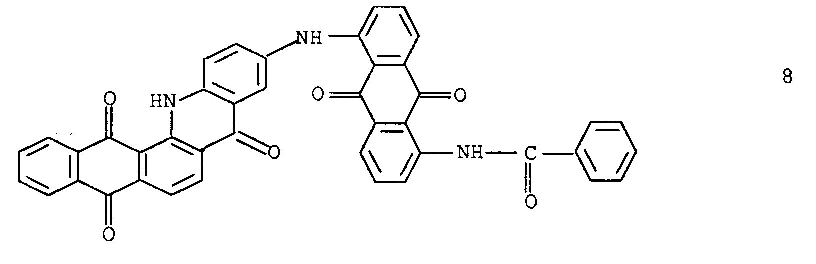
Anschließende Carbazolierung in Schwefelsäure lieferte den entsprechenden braunen Küpenfarbstoff in guter Qualität.

### Beispiel 9

Ein Gemisch aus 890 g Benzoesäuremethylester, 106 g Dibromfluoranthen, 152 g 1-Amino-4-benzoylaminoanthrachinon, 223 g wasserfreiem Trinatriumphosphat und 15,3 g Kupferpulver wurde unter Rühren 24 h auf 175°C erhitzt.

Nach Wasserdampfdestillation zur Entfernung des Lösungsmittels, Filtrieren und Trocknen wurden 245 g des Anthrimids der Formel 9 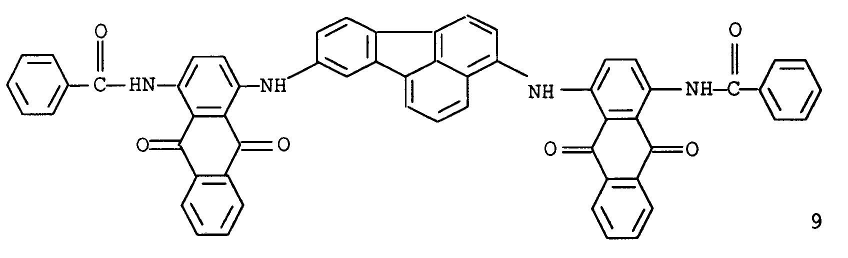
erhalten.

Carbazolierung in Schwefelsäure ergab den entsprechenden braunen Küpenfarbstoff in guter Qualität.

### Beispiel 10

Ein Gemisch aus 240 g Benzoesäuremethylester, 10 g Tetrabrompyranthron, 12,9 g Aminoviolanthron, 6,7 g 1-Aminoanthrachinon, 5,6 g wasserfreiem Natriumcarbonat, 2,7 g Kupfer(I)oxid und 4 g m-Nitrobenzolsulfonsäurenatriumsalz wurde unter Rühren 20 h auf 200°C erhitzt.

Nachfolgende Wasserdampfdestillation, Filtrieren und Trocknen ergaben 27,6 g des Küpenfarbstoffs C.I. Vat Black 9 (Formel 10)

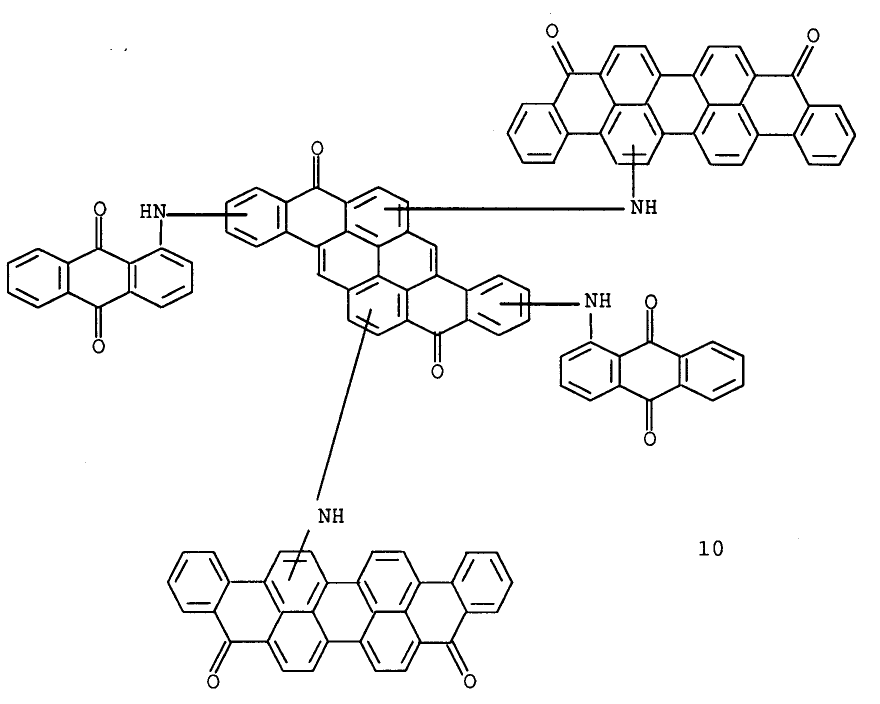

### Beispiel 11

Ein Gemisch aus 1300 g Benzoesäuremethylester, 103,6 g in Schwefelsäure bromiertem Dibenzanthron (Bromgehalt 27,8 Gew.-%), 80,3 g 1-Aminoanthrachinon, 57,3 g wasserfreiem Natriumcarbonat und 25 g Kupfer(I)chlorid wurde unter Rühren 20 h auf Rückflußtemperatur erhitzt.

Nach Entfernung des Lösungsmittels durch Wasserdampfdestillation wurde der Farbstoff abfiltriert, durch dreistündiges Erhitzen in 2500 ml 10 gew.-%iger Salzsäure unter Rückfluß entkupfert, erneut abfiltriert und getrocknet.

Es wurden 151,3 g des schwarzen Küpenfarbstoffs der Formel 11 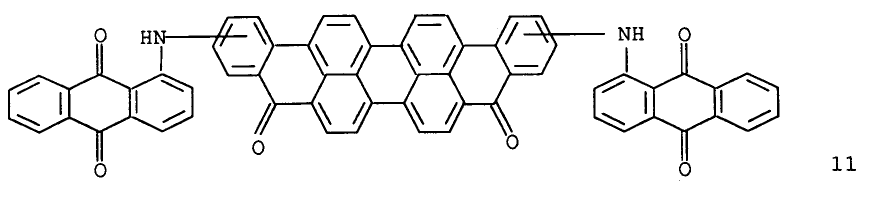
erhalten.

## Patentansprüche

1. Verfahren zur Herstellung von Anthrachinonaminverbindungen durch Kondensation von mindestens eine primäre Aminogruppe enthaltenden Anthrachinonverbindungen mit halogenierten Aromaten oder von halogenierten Anthrachinonverbindungen mit primären aromatischen Aminen in Gegenwart eines Kupferkatalysators und eines säurebindenden Mittels in einem organischen Lösungsmittel, dadurch gekennzeichnet, daß man als organisches Lösungsmittel einen Benzoesäurealkylester verwendet.

2. Verfahren nach Anspruch 1, dadurch gekennzeichnet, daß man als organisches Lösungsmittel Benzoesäuremethylester verwendet.

3. Verfahren nach Anspruch 1 oder 2, dadurch gekennzeichnet, daß man die Kondensation in Gegenwart eines Oxidationsmittels durchführt.

4. Verfahren nach den Ansprüchen 1 bis 3, dadurch gekennzeichnet, daß man es zur Herstellung von Anthrachinonaminverbindungen der allgemeinen Formel I 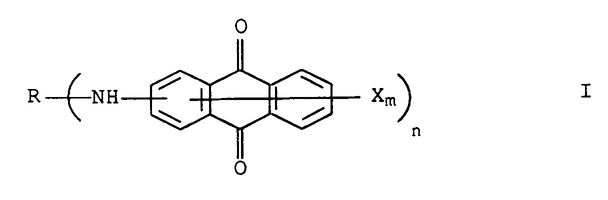 anwendet, in welcher die Variablen folgende Bedeutung haben:
R einen Phenylrest, einen Anthrachinon-, Benzanthron-, Anthanthron-, Pyranthron-, Dibenzanthron-, Isodibenzanthron-, Anthrachinonacridon-, Flavanthron-, Fluoranthen-, Dibenzpyrenchinonrest oder den Rest eines Kondensationsprodukts dieser Aromaten, wobei die genannten Reste für Küpenfarbstoffe übliche Substituenten tragen können;
X Halogen, Hydroxy, Amino, C₁-C₄-Alkylamino, C₁-C₄-Alkanoylamino, Benzoylamino, dessen Benzoylrest durch Halogen oder C₁-C₄-Alkyl substituiert sein kann, oder C₁-C₄-Alkyl;
m 0 bis 4, wobei die Reste X für m ≧ 2 gleich oder verschieden sein können;
n 1 bis 4.

## Claims

1. A process for preparing anthraquinone-amine compounds by condensation of anthraquinone compounds which contain at least one primary amino group with halogenated aromatic compounds or of halogenated anthraquinone compounds with primary aromatic amines in the presence of a copper catalyst and of an acid acceptor in an organic solvent, wherein an alkyl benzoate is used as organic solvent.

2. A process as claimed in claim 1, wherein methyl benzoate is used as organic solvent.

3. A process as claimed in claim 1 or 2, wherein the condensation is carried out in the presence of an oxidizing agent.

4. A process as claimed in any of claims 1 to 3, which is used to prepare anthraquinone-amine compounds of the general formula I 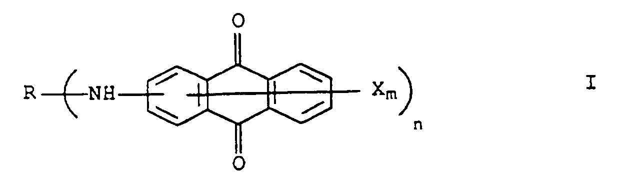 in which the variables have the following meaning:
R a phenyl radical, an anthraquinone, benzanthrone, anthanthrone, pyranthrone, dibenzanthrone, isodibenzanthrone, anthraquinoneacridone, flavanthrone, fluoranthene, dibenzpyrenequinone radical or the radical of a condensate of these aromatic compounds, where the said radicals can carry substituents customary for vat dyes;
X halogen, hydroxyl, amino, C₁-C₄-alkylamino, C₁-C₄-alkanoylamino, benzoylamino whose benzoyl radical can be substituted by halogen or C₁-C₄-alkyl, or C₁-C₄-alkyl;
m 0 to 4, where the X radicals can be identical or different when m ≧ 2;
n 1 to 4.

## Revendications

1. Procédé de préparation d'anthraquinone-amines par condensation de composés d'anthraquinone contenant au moins un groupement amino primaire avec des composés aromatiques halogénés ou de composés d'anthraquinone halogénés avec des amines primaires aromatiques en présence d'un catalyseur au cuivre et d'un agent fixant les acides dans un solvant organique, caractérisé en ce que l'on utilise, comme solvant organique, un benzoate d'alkyle.

2. Procédé selon la revendication 1, caractérisé en ce que l'on utilise, comme solvant organique, du benzoate de méthyle.

3. Procédé selon la revendication 1 ou 2, caractérisé en ce que l'on effectue la condensation en présence d'un oxydant.

4. Procédé selon l'une quelconque des revendications 1 à 3, caractérisé en ce qu'on l'applique pour la préparation d'anthraquinone-amines de formule générale I 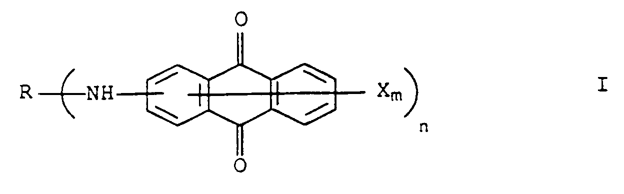 dans laquelle les variables ont les significations suivantes:
R reste phényle, reste anthraquinone, benzanthrone, anthanthrone, pyranthrone, dibenzanthrone, isodibenzanthrone, antbraquinone-acridone, flavanthrone, fluoranthène, dibenzopyrènequinone ou le reste d'un produit de condensation de ces substances aromatiques, lesdits restes pouvant porter des substituants usuels pour des colorants de cuve;
X atome d'halogène, groupement hydroxy, amino, alkylamino en C₁-C₄, alcanoylamino en C₁-C₄, benzoylamino dont le reste benzoyle peut être substitué par un atome d'halogène ou un reste alkyle en C₁-C₄, ou groupement alkyle en C₁-C₄;
m 0 à 4, les restes X pouvant être identiques ou différents lorsque m ≧ 2;
n 1 à 4.
